# EUROPEAN PATENT APPLICATION

(11) **EP 4 799 606 A1**
(43) Date of publication of application: **02.09.2026**
(21) Application number: 24882037.5
(22) Date of filing: 06.09.2024
(51) Int. Cl.: A61K 8/99, A61K 8/44, A61K 8/60, A61K 31/198, A61K 31/7028, A61P 17/00, A61P 17/18, A61P 29/00, A61Q 17/04, A61Q 19/00

(54) **MYCOSPORINE-CONTAINING COMPOSITION**

(30) Priority: 26.10.2023 JP 2023184292
(71) Applicant: Nissui Corporation, Tokyo 105-8676 (JP)
(72) Inventor: NAKAJIMA, Toshiaki, Hachioji-shi, Tokyo 192-0991 (JP)
(74) Representative: Mewburn Ellis LLP
(86) International application number: PCT/JP2024/032064
(87) International publication number: WO 2025/088920

(57) **Abstract**

The present disclosure aims to provide a composition that can be used as an alternative to synthetic ultraviolet absorbers and that exhibits an excellent skin-problem-suppressing effect by means of a stable component that absorbs ultraviolet over a wide wavelength range. Mycosporines (MYCs) are used as an active ingredient of a composition for suppressing a skin problem and/or a composition for suppressing reactive oxygen species. The MYCs preferably include one, or two or more, selected from mycosporine-glutaminol-glucoside (MGGnol), mycosporine-glutamicol-glucoside (MGGcol), and mycosporine-glutamine (MGn). The skin problem may be a skin problem caused by ultraviolet irradiation, or may be a skin problem caused without ultraviolet irradiation.

## Description

### TECHNICAL FIELD

The present disclosure relates to a composition comprising a mycosporine (MYC) and provides a novel use of MYCs.

### BACKGROUND ART

When skin is exposed to ultraviolet, various undesirable conditions (skin problems) such as inflammation and pigmentation occur. Therefore, sunscreen cosmetics formulated with an ultraviolet absorber or an ultraviolet-scattering agent are widely used.

Many of the ultraviolet absorbers currently used in sunscreen cosmetics are synthetic, and those covering UV-B and UV-A are common. There have been concerns that synthetic ultraviolet absorbers may cause problems such as generation of reactive oxygen species by photolysis, induction of allergy and itching, and environmental pollution (Non-Patent Document 1, Non-Patent Document 2).

For these reasons, there is a demand for natural ultraviolet absorbers, and their development has been promoted.

Among natural ultraviolet absorbers, mycosporine-like amino acids (MAAs) are naturally occurring substances known to efficiently absorb ultraviolet such as UV-A and UV-B. MAAs are expected to be applied as ultraviolet absorbers to be included in sunscreen cosmetics and the like.

For example, MAAs (including porphyra-334 and shinorine) extracted from red algae have already been commercialized (Non-Patent Document 3). However, since production of such MAAs tends to be affected by climate and the like and hence unstable, it is difficult to stably obtain a large amount of MAAs.

Although a method of producing shinorine by a genetic recombination technique has been proposed (Patent Document 1), some consumers feel reluctant to accept genetic recombination techniques, and such products have not yet been commercialized.

Further, while MAAs whose ultraviolet-absorbing capacities are expected, such as shinorine, have absorption wavelengths in the UV-A2 range (320 to 340 nm), they do not cover the UV-B and UV-A1 wavelength ranges (340 to 400 nm). Therefore, such MAAs are insufficient as ultraviolet absorbers (Non-Patent Document 1). In particular, Non-Patent Document 2 points out that conventional MAAs do not show effects as ultraviolet absorbers. In addition, porphyra-334 is sensitive to heat (Non-Patent Document 4), and shinorine undergoes coloration during storage (Patent Document 2). Therefore, these have problems with the stability, and hence quality issues prevent their commercialization.

Various actions of MAAs have been reported, and examples of such actions that have been inferred include antioxidant action, suppression of DNA damage, suppression of lipid peroxidation, and suppression of advanced glycation end products. On the other hand, MAAs have been reported to have no tyrosinase inhibitory action, which is involved in pigmentation (Non-Patent Document 5).

These actions have been evaluated in cell-based tests and mouse tests, but have hardly been evaluated in human tests (Non-Patent Document 2).

Meanwhile, various functionalities of mycosporines (MYCs), which are similar to MAAs (and are sometimes treated as belonging to the same group as MAAs), have also been studied. "MYCs" is a general term for compounds that have a cyclohexenone or cyclohexenimine skeleton, have an ultraviolet-absorbing capacity, and are biosynthesized. The compounds that are "biosynthesized" may include, for example, compounds that are produced by aquatic organisms such as algae and shellfish or by microorganisms such as yeasts and molds, or compounds that can be obtained from these organisms. Specific examples of such compounds include mycosporine-glutaminol-glucoside (MGGnol), mycosporine-glutamicol-glucoside (MGGcol), mycosporine-glutamine (MGn), mycosporine-glutamic acid (MGa), mycosporine-glycine (MG), and Collemin A.

MG and Collemin have been reported to have various actions on the skin, for example, an erythema-suppressing action, a fibroblast proliferation action, and a damaged-skin healing action. However, the reliability of these effects is low, and useful effects attributable to ultraviolet-absorbing capacity have not been demonstrated (Non-Patent Documents 6 and 7, Patent Document 3). In addition, as with MAAs, no method has been available for mass production of MYCs, and MYCs have problems with the stability (Non-Patent Document 8).

With respect to MGGnol, it has been reported that epidermal cells to which MGGnol was added showed an increased survival rate upon UV-B irradiation, that MGGnol has actions such as a fibroblast proliferation action, a collagen productionpromoting action, and, in epidermal cells, a prostaglandin E₂ (PGE₂) productionsuppressing action, and that MGGnol quenches singlet oxygen (Patent Document 4, Non-Patent Document 9). However, since keratin is absent in epidermal cells, it cannot be said that the actions and effects that actually occur upon application of the compounds to human skin have been correctly evaluated.

### PRIOR ART DOCUMENTS

### [Patent Documents]

[Patent Document 1] JP 5927593 B
[Patent Document 2] JP 6719267 B
[Patent Document 3] JP 2007-16004 A
[Patent Document 4] JP 5913988 B

### [Non-patent Documents]

[Non-Patent Document 1] Tadashi Mori, Annual Report of Cosmetology vol. 27, 64-67 (2019)
[Non-Patent Document 2] K. P. Lawrence, et al., Current Medical Chemistry 25, 5512-5527 (2018)
[Non-Patent Document 3] S. Daniel, et al., Cosmetic and Toiletries Manufacture worldwide, 139-143 (2004)
[Non-Patent Document 4] M. Yoshiki et al., Food Chemistry, 113, 1127-1132 (2009)
[Non-Patent Document 5] K. Hakuto et al., Marine Drugs, 14, 222, 120-137 (2019)
[Non-Patent Document 6] A. Torres, et al., Eur. J. Biochem 271, 780-784 (2004)
[Non-Patent Document 7] H. J. Suh, et al., Photochem photobiol 78(2), 109-113 (2003)
[Non-Patent Document 8] S. Ito, et al., Tetrahedron Lett, 28, 2429-2430 (1977)
[Non-Patent Document 9] M. Moline, et al., RADIATION RESEARCH 175, 44-50 (2011)

### SUMMARY OF THE INVENTION

### PROBLEMS TO BE SOLVED BY THE INVENTION

In view of such circumstances, an object of the present disclosure is to provide a composition that can be used as an alternative to synthetic ultraviolet absorbers and that exhibits an excellent skin-problem-suppressing effect by means of a stable component that absorbs ultraviolet over a wide wavelength range.

### MEANS FOR SOLVING THE PROBLEMS

Previously, the inventors of the present disclosure invented a method of producing MYCs by culturing a black yeast, in which MYCs are produced in a large amount and efficiently extracted (Japanese Patent Application No. 2023-2010, Japanese Patent Application No. 2023-2008, Japanese Patent Application No. 2023-2009). These inventions led to the expectation that industrial production of MYCs may be possible, and the commercialization of MYCs has become realistic.

In the course of intensive studies to solve the above problem, the inventors of the present disclosure focused on the possibility of industrial production of the above MYCs and evaluated the functionality of MYCs using human skin sections, three-dimensional human skin models, and the like. As a result, the inventors discovered that MYCs suppress skin problems caused by a variety of mechanisms and hence can serve as an active ingredient of compositions for suppressing skin problems. The actions and effects of MYCs discovered by the inventors of the present disclosure are described below.

The inventors of the present disclosure discovered that MYCs absorb UV-B to suppress erythema and inflammation, to suppress pigmentation such as darkening and spots, and to suppress generation of reactive oxygen species (ROS) and hence suppress oxidative stress. Carbonyl protein (CP) is a footprint of oxidative stress. In other words, generation of CP indicates a state with exposure to oxidative stress. Oxidative stress is involved in wrinkles, spots, skin aging, and the like. By suppression of oxidative stress, these are prevented. Generation of CP causes discoloration of skin to yellowish brown and skin dryness, and irradiation of CP with UV-A causes generation of reactive oxygen species, so that suppression of CP is of great significance.

Further, although the inventors of the present disclosure considered that the skin-problem-suppressing effect of MYCs was an effect due to absorption of UV-B by MYCs, particularly MGGnol, MGGcol, and MGn, the inventors surprisingly discovered that the above effect of the MYCs can be obtained also by suppression of oxidative stress through a pathway in which singlet oxygen, a type of reactive oxygen species generated by UV-A, is absorbed.

Furthermore, the inventors of the present disclosure also discovered that, even in a state without ultraviolet irradiation, MYCs have an anti-inflammatory action and an active-oxygen-scavenging action, and therefore suppress skin inflammation and oxidative stress.

Specifically, the present disclosure includes the following inventions. The term "MYCs" may include a single type or a plurality of types of mycosporines.
[1] A composition for suppressing a skin problem, the composition comprising a mycosporine (MYC).
[2] A composition for suppressing reactive oxygen species, the composition comprising a mycosporine (MYC).
[3] The composition according to [1] or [2], wherein the MYC comprises one, or two or more, selected from mycosporine-glutaminol-glucoside (MGGnol), mycosporine-glutamicol-glucoside (MGGcol), and mycosporine-glutamine (MGn).
[4] The composition according to any one of [1] to [3], wherein the MYC comprises at least MGGnol and MGGcol.
[5] The composition according to any one of [1] to [3], comprising MGGnol, MGGcol, and MGn in a total amount of not less than 0.001% by weight based on the entire composition.
[6] The composition according to any one of [1] to [5], which is used by application to skin in a total amount of MGGnol, MGGcol, and MGn of not less than 0.1 µg/cm²/day.
[7] The composition according to [1], wherein the skin problem is one or more selected from erythema, inflammation, darkening/spots, and phenomena caused by oxidative stress, in skin.
[8] The composition according to [1], wherein the skin problem is a skin problem caused by ultraviolet irradiation, or a skin problem caused without ultraviolet irradiation.
[9] The composition according to any one of [1] to [8], which is a topical skin composition.
[10] The composition according to [9], which is used by application to skin in a total amount of MGGnol, MGGcol, and MGn of not less than 0.1 µg/cm²/day.
[11] The composition according to [9], which is for suppressing erythema and/or inflammation in skin and is used by application to skin in a total amount of MGGnol, MGGcol, and MGn of not less than 0.1 µg/cm²/day.
[12] The composition according to [9], which is for suppressing darkening and/or spots in skin and is used by application to skin in a total amount of MGGnol, MGGcol, and MGn of not less than 0.5 µg/cm²/day.
[13] The composition according to [9], which is for suppressing a phenomenon caused by oxidative stress in skin and is used by application to skin in a total amount of MGGnol, MGGcol, and MGn of not less than 0.1 µg/cm²/day.
[14] The composition according to [9], which is a sunscreen cosmetic.

### EFFECT OF THE INVENTION

The present disclosure provides a composition that exhibits an excellent suppressive effect on various skin problems caused by ultraviolet irradiation, as well as on various skin problems caused without ultraviolet irradiation. Since the composition suppresses skin inflammation and the amount of ROS in skin even in a steady state without ultraviolet irradiation, the present disclosure is advantageous from the viewpoint of the fact that not only countermeasures against ultraviolet, but also an effect of suppressing various common skin problems can be expected.

The present disclosure also provides a composition for suppressing reactive oxygen species (ROS). Since reactive oxygen species is a factor that causes oxidative stress, its suppression is advantageous.

In addition, MYCs such as MGGnol, MGGcol, and MGn are usually watersoluble and do not disappear by degradation or the like even through hightemperature conditions, so that they can be subjected to sterilization treatment by autoclaving. Furthermore, unlike common synthetic ultraviolet absorbers, MYCs do not undergo photolysis, and therefore do not generate reactive oxygen species. Therefore, they can preferably be used as an alternative to synthetic ultraviolet absorbers. Thus, MYCs are suitable for blending into compositions such as topical skin compositions including cosmetics. In particular, since blending of preservatives and synthetic ultraviolet absorbers in the compositions can be reduced, the compositions can meet the needs of users who are hypersensitive to chemical substances.

### BRIEF DESCRIPTION OF THE DRAWING

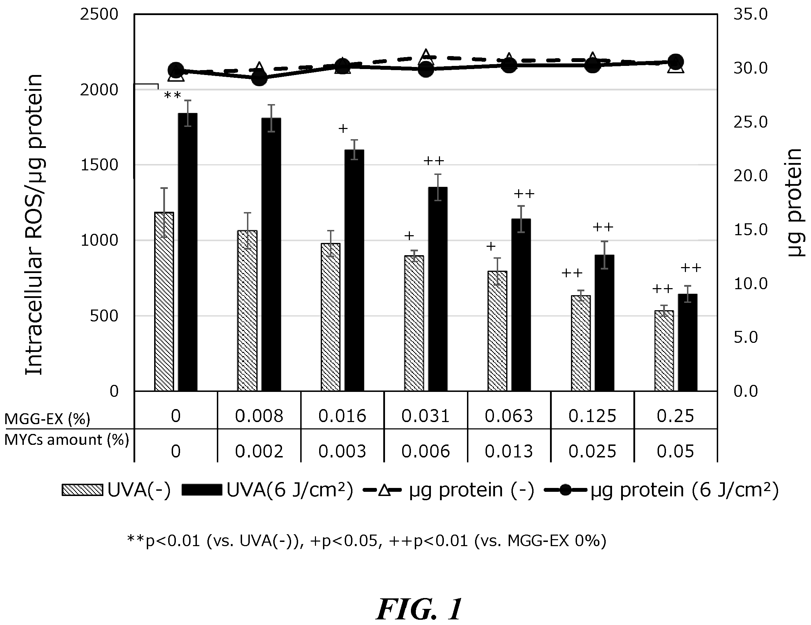

Fig. 1 is a graph illustrating the ROS level in cells to which MYCs were applied in Example 5, as observed for cases with or without UV-A irradiation.

### MODE FOR CARRYING OUT THE INVENTION

The composition of the present disclosure indispensably comprises an MYC as an active ingredient. In a preferred embodiment, the MYC includes one, or two or more, selected from MGGnol, MGGcol, and MGn, and in a more preferred embodiment, the MYC includes at least MGGnol and MGGcol.

The MYC may be obtained, for example, as a commercial product, or may be produced by an arbitrary method from a microorganism that produces the MYC.

In a specific mode of a production means, the MYC can be obtained by culturing a microorganism that produces the MYC and extracting the MYC from the microorganism.

The microorganism as the extraction source may be of a single strain, or may be of a plurality of strains.

The microorganism that produces the MYC is not particularly limited. Examples of a microorganism that produces MGGnol and/or MGGcol include a black yeast that belongs to the family Dothioraceae of the order Dothideales, and that produces MGGnol and/or MGGcol. In general, yeasts having a black color are referred to as black yeasts, and examples of black yeasts include, in addition to those classified in the family Dothioraceae of the order Dothideales, those belonging to the genus *Moniliella* of the phylum Basidiomycota or to the genus *Exophiala* of the family Ferpotrichiellaceae of the order Chaetothyriales (de Hoog et al., Atlas of Clinical Fungi, 2nd edition: Utrecht: centraalubureauvoor Schimmelcultures; (2000)). However, the black yeast referred to herein may be a yeast classified in the family Dothioraceae of the order Dothideales. Specifically, the black yeast may be a black yeast belonging to the genus *Aureobasidium*, the genus *Hormonema*, or the genus *Hortaea*.

Here, the black yeast belonging to the genus *Hormonema* may include those whose teleomorph belongs to the genus *Sydowia*. The anamorph of the genus *Sydowia* corresponds to the genus *Hormonema* and differs only in whether reproduction is sexual or asexual in the same fungal strain (N.A. Yurlava et al., Studies in Mycology., 43, 63-69 (1999); de Hoog et al., Atlas of clinical fungi, 2nd edition: Utrecht: centraalubureauvoor Schimmelcultures; (2000)). Cells described as *Sydowia* cells sometimes grow into cells with a yeast-like morphology when the cells are subjected to liquid culture. Since those cells can be identified as the genus *Hormonema*, which is the anamorph (G.S. de Hoog et al., Antonie van Leeuwenhoek., 65, 41-54 (1994)), both are described in an inclusive manner in the present description.

Although yeasts whose anamorph belongs to the genus *Aureobasidium* and yeasts whose anamorph belongs to the genus *Hormonema* are closely related to each other, these yeasts are clearly distinguished by molecular phylogeny (Non-Patent Document 7).

Specific examples of the black yeasts of the family Dothioraceae of the order Dothideales that produce MGGnol and/or MGGcol are not particularly limited, and any strain of such black yeasts may be used.

Examples of the black yeasts of the genus *Aureobasidium* include *Aureobasidium pullulans*, *Aureobasidium subglaciale*, *Aureobasidium aerium*, *Aureobasidium microstictum*, and *Aureobasidium proteae*. Specific examples of the fungal strains of *Aureobasidium pullulans* include the NRBC 6353 strain, the NRBC 100716 strain, the NRBC 106987 strain, the NRBC 4464 strain, the NRBC 7757 strain, NRBC 108784, and the NRBC 114573 strain (all of which are available from the Patent Microorganisms Depositary (NPMD), National Institute of Technology and Evaluation (address: Room 122, 2-5-8 Kazusakamatari, Kisarazu-shi, Chiba 292-0818, Japan)). The IDF-23 strain (NPMD-deposited strain; accession number, FERM P-22141) described in Patent Document 1 may also be used.

Examples of the black yeasts of the genus *Hormonema* include *Hormonema macrosporum* and *Hormonema dematioides*. Specific examples of the fungal strains whose anamorph corresponds to *Hormonema macrosporum* and which are distributed as *Sydowia polyspora* (teleomorph) include the NRBC 107026 strain, the NRBC 107027 strain, the NRBC 107028 strain, the NRBC 5982 strain, and the NRBC 32065 strain (all of which are available from NPMD).

Examples of the black yeasts of the genus *Hortaea* include *Hortaea wirneckii*. Specific examples of the fungal strains of *Hortaea wirneckii* include the NRBC 4875 strain and the NRBC 6407 strain (both of which are available from NPMD).

Examples of the microorganisms that produce MGn include, but are not limited to, fungi that belong to the genus *Hormonema* and that produce MGn. Examples of the fungi belonging to the genus *Hormonema* include *Hormonema macrosporum* and *Hormonema dematioides*. Specific examples of the fungal strains of *Hormonema macrosporum* include the NSK102 strain (NITE ABP-03776), the NSK33 strain (NITE ABP-03775), and the NSK6 strain (NITE ABP-03774).

Each of the NSK102 strain (NITE ABP-03776), the NSK33 strain (NITE ABP-03775), and the NSK6 strain (NITE ABP-03774) of *Hormonema macrosporum* is a new fungus isolated from a natural source (Japanese Patent Application No. 2023-2008). When these fungi were subjected to liquid culture, and the shapes of their cells were observed under a light microscope, yeast-type cells were found. In order to investigate the genetic characteristics of these fungi, the base sequence of the D1/D2 region of 28S rRNA was identified by an ordinary method. Further, the base sequence of the 28S rRNA gene of each *Hormonema macrosporum* strain was subjected to homology search against DB-FU15.0 (Techno Suruga Laboratory, Japan) and international base sequence databases (DDBJ/ENA/GenBank) (search date, July 29, 2022) by BLAST analysis using the analysis software ENKI v3.2 (Techno Suruga Laboratory, Japan). In the analysis, a phylogenetic tree was estimated by the neighbor-joining method. The Kimura-2-parameter was used as a base substitution model, and the reliability of the topology was evaluated by the bootstrap method (1000 replications). As a result, all three strains were identified as *Hormonema macrosporum* (anamorph) or *Sydowia polysopra* (teleomorph).

The NSK102 strain of *Hormonema macrosporum* has been the subject of an application for international deposition with Patent Microorganisms Depositary, National Institute of Technology and Evaluation (Address: Room 122, 2-5-8 Kazusakamatari, Kisarazu-shi, Chiba, 292-0818 Japan) under the accession No. NITE ABP-03776 under the Budapest Treaty (receiving date: October 20, 2022).

The NSK33 strain of *Hormonema macrosporum* has been the subject of an application for international deposition with Patent Microorganisms Depositary, National Institute of Technology and Evaluation (Address: Room 122, 2-5-8 Kazusakamatari, Kisarazu-shi, Chiba, 292-0818 Japan) under the accession No. NITE ABP-03775 under the Budapest Treaty (receiving date: October 20, 2022).

The NSK6 strain of *Hormonema macrosporum* has been the subject of an application for international deposition with Patent Microorganisms Depositary, National Institute of Technology and Evaluation (Address: Room 122, 2-5-8 Kazusakamatari, Kisarazu-shi, Chiba, 292-0818 Japan) under the accession No. NITE ABP-03774 under the Budapest Treaty (receiving date: October 20, 2022).

An example of a method of producing MYCs from a microorganism is described below, but the method of obtaining MYCs is not limited thereto.

The medium used in the culture step of culturing a microorganism producing MYCs is not limited as long as the medium sufficiently allows the growth of the microorganism, and may contain a carbon source, a nitrogen source, an inorganic salt, and, when necessary, components such as amino acids suitable for the growth of the microorganism. Examples of the carbon sources that may be used include sugars such as glucose, starch, and waste molasses; organic acids; and sodium acetate; especially sugars such as glucose. Examples of the nitrogen sources that may be used include ammonium salts, yeast extract, corn steep liquor, and peptone. Examples of the inorganic salts that may be used include magnesium salts, calcium salts, phosphate salts, iron salts, and copper salts. Usually, a liquid medium prepared by mixing and dissolving these components in water may be used.

The pH may usually be within the range of 2 to 9, 3 to 8, or 3.5 to 7.5. The growth of the microorganism may be inhibited in cases where the pH is too high or too low.

In the culture step, the culture temperature is not limited as long as it is a condition that sufficiently allows the growth of the microorganism. The culture temperature may be, for example, 15 to 40°C, 18 to 35°C, or 20 to 30°C. In cases where the culture temperature is too low, the growth rate is insufficient, while in cases where the culture temperature is too high, biosynthetic pathways tend to be abnormal.

In the culture step, the culture period is not limited. The culture may be carried out for 72 hours to 240 hours, or 96 hours to 168 hours.

Further, in the culture step, light irradiation may or may not be carried out.

After the culture step, an extraction step of extracting an MYC fraction from the MYC-producing microorganism into a solvent is carried out.

Here, in the extraction, the efficiency of extraction tends to be improved when the following (a) and/or (b) is/are satisfied. However, in cases where the following (c) is satisfied in the culture step, efficient extraction is possible even without carrying out a step satisfying (a) or (b). In other words, the method may satisfy one or more selected from the following (a), (b), and (c).
(a) In the extraction step, an MYC fraction is extracted into a solvent at not less than 40°C.
(b) Before the extraction step, a heating step of collecting the microorganism from the culture liquid after the culture step, and heating the collected microorganism, is carried out.
(c) In the culture step, the culture is carried out in a medium containing glucose and yeast extract, and/or carried out without light irradiation.

After the culture step, but before the extraction step, microorganism cells may be collected from the culture liquid. The collection of the microorganism cells may be carried out by centrifugation, filtration, or the like. The collected microorganism cells may be dried using a heat dryer, a freeze dryer, a vacuum dryer, a flash dryer, or the like.

In the production method, in the (a) extraction of the MYC fraction into the solvent at not less than 40°C, the microorganism is brought into contact with the extraction solvent.

As the extraction solvent, a polar solvent may be used, or a polar solvent containing mainly water may be used. Examples of such a polar solvent include water; and polar organic solvents such as lower alcohols (including methanol, ethanol, butyl alcohol, isopropanol, and butylene glycol) and acetone. Examples of the polar solvent containing mainly water include water; and aqueous solutions of a polar organic solvent such as a lower alcohol (for example, methanol, ethanol, butyl alcohol, isopropanol, or butylene glycol) or acetone. Water alone may also be used. As the solvent "containing mainly water", a solvent containing water at not less than 80% by weight, not less than 85% by weight, or not less than 90% by weight in the entire extraction solvent may be used. The higher the water content in the extraction solvent, the higher the efficiency of extraction can be expected to be. In other words, in cases where an aqueous solution of a polar organic solvent, such as a lower alcohol, is used as the extraction solvent, the concentration of the polar organic solvent may not be more than 20% by weight, or the organic solvent may not be substantially used. Here, "the organic solvent is not substantially used" means that the concentration of the polar organic solvent in the aqueous solution used as the extraction solvent is not more than 1% by weight, not more than 0.1% by weight, or not more than 0.01% by weight. In cases where the polar organic solvent is not substantially used in the extraction, the step of evaporating the polar organic solvent from the obtained extract can be eliminated.

The temperature of the extraction solvent in the extraction of the MYC fraction into the solvent is 40°C to 95°C, or may be 50°C to 90°C, or 60°C to 85°C. In cases where the temperature of the extraction solvent is too low, the extraction efficiency tends to decrease. In cases where the temperature of the extraction solvent is too high, a problem tends to occur in the stability of a desired component. Further, an apparatus such as an autoclave may be used to perform heating to not less than 90°C, or to a temperature of 100°C to 135°C, 110°C to 130°C, or 120°C to 125°C. The heating can inhibit the actions of undesired enzymes contained in the cells. In cases where the heating temperature is too low, the inhibition of the actions of undesired enzymes may be insufficient, while in cases where the heating temperature is too high, a problem tends to occur in the stability of a desired component. Further, pressurization may be carried out to not less than 0.10 MPa, not less than 0.12 MPa, not less than 0.15 MPa, or not less than 0.20 MPa. The pressurization promotes the inhibition of the actions of undesired enzymes contained in the cells. In cases where the pressurization is insufficient, the inhibition of the actions of undesired enzymes contained in the cells may be insufficient. From the viewpoint of safety, the pressurization may be carried out at not more than 1.00 MPa.

The extraction time may be 15 minutes to 120 minutes, 60 minutes to 110 minutes, or 90 minutes to 100 minutes. The longer the extraction time, the more likely the extracted amount is to be increased. The upper limit of the extraction time may be, for example, about 120 minutes. In cases where the extraction time is too long, undesired impurities are likely to be contained. During the extraction step, the microorganism and the extraction solvent that have been brought into contact with each other may be stirred or shaken as appropriate, or may be left to stand.

After the culture step, the culture liquid may be subjected, directly or after being diluted as appropriate, to the extraction step without collecting the microorganism from the culture liquid, to carry out the (a) extraction of the MYC fraction into the solvent at not less than 40°C. In this case, the culture liquid may be heated to not less than 40°C or not less than 50°C. Efficient extraction can be expected by the heating of the culture liquid in advance. Usually, the culture liquid contains a polar solvent, especially water.

The culture liquid after the culture step may be diluted, for example, not less than 2-fold by volume with water, a buffer, a medium, or the like before being subjected to the extraction step. In cases where the culture liquid is diluted with water, a buffer, a medium, or the like before its use in the extraction step, the culture liquid can be more easily handled. For example, in cases where the viscosity of the culture liquid is high, the culture liquid may be diluted with the buffer, medium, or the like before its use in the extraction step. By this, retention of the culture liquid in the apparatus can be reduced in some cases. The culture liquid may also be used directly in the extraction step. By the direct use of the culture liquid, the extraction cost may be reduced.

In the production method disclosed herein, the (b) heating step of heating, before the extraction step, the microorganism collected from the culture liquid after the culture step may be carried out instead of or prior to the (a). In this step, under the following conditions, the microorganism can be damaged or broken to improve the extraction efficiency of MYCs, or the actions of undesired enzymes in the microorganism cells can be inhibited to improve the amount of MYCs recovered.

The heating of the microorganism in the heating step may be carried out under conditions selected from the following (i) temperature, (ii) length of time, and (iii) pressure.
(i) The temperature to which the microorganism is exposed may be 40°C to 200°C, 50°C to 190°C, 60°C to 180°C, 70°C to 170°C, 80°C to 160°C, 90°C to 150°C, 100°C to 140°C, 105°C to 130°C, 110°C to 130°C, 115°C to 130°C, 120°C to 130°C, or 125°C to 130°C. In cases where the temperature to which the microorganism is exposed is too low, the inhibition of the actions of undesired enzymes may be insufficient, while in cases where the temperature to which the microorganism is exposed is too high, a problem tends to occur in the stability of a desired component.
(ii) The length of time for which the microorganism is heated may be 2 to 6 hours, 3 to 5 hours, or 4 to 5 hours. The heating time may be a time for which a certain heating temperature is maintained after the heating temperature is achieved. In cases where the length of time for which the microorganism is heated is too short, the inhibition of the actions of undesired enzymes may be insufficient, while in cases where the length of time for which the microorganism is heated is too long, a problem tends to occur in the stability of a desired component.
(iii) The pressure applied to the microorganism, calculated as the average pressure during the period when the microorganism is exposed to heating, may be 0.01 MPa to 0.10 MPa, 0.02 MPa to 0.08 MPa, or 0.03 MPa to 0.05 MPa. In cases where the pressure applied to the microorganism is too low, the inhibition of the actions of undesired enzymes may be insufficient, while in cases where the pressure applied to the microorganism is too high, a problem tends to occur in the stability of a desired component.

The conditions (i) to (iii) may be arbitrarily selected and combined. In particular, at least (i) and (ii) may be combined. In this case, by heating at a high temperature for a certain length of time, sterilization can be achieved at the same time so that enzymes in the microorganism can be inactivated. Therefore, the amount of MYCs recovered can be expected to be further improved. In addition, since the microorganism is broken by (iii), the extraction efficiency of MYCs may be further improved. The heating step may be carried out under conditions where the microorganism is dried. For example, the heating step may be carried out under one or more conditions selected from (i) a temperature of not less than 105°C, (ii) a heating time of not less than 2 hours, and (iii) a pressure of not more than 0.10 MPa. The heating step may also be carried out under the conditions of (i) a temperature of not less than 105°C, (ii) a heating time of not less than 2 hours, and (iii) a pressure of not more than 0.10 MPa.

In the production method disclosed herein, in cases where the extraction step is carried out using a microorganism that has been subjected to the (b) heating step, the extraction step may be carried out under the conditions described in (a), or may be carried out at a low extraction temperature.

Specifically, the extraction solvent used may be the same as that described in (a). On the other hand, the temperature of the extraction solvent during the extraction of the MYC fraction into the solvent may not be less than 5°C, may not be less than 10°C, or may not be less than 25°C (normal temperature). The conditions described in (a) are also applicable. Thus, the temperature may be 40°C to 70°C, 50°C to 90°C, or 60°C to 100°C, or an apparatus such as an autoclave may be used to perform heating to a temperature of 90°C to 150°C, 100°C to 140°C, 110°C to 130°C, or 120°C to 125°C.

The extraction time may not be less than 15 minutes, not less than 60 minutes, or not less than 90 minutes. The longer the extraction time, the more likely the extracted amount is to be increased. The upper limit of the extraction time may be, for example, about 120 minutes. During the extraction step, the microorganism and the extraction solvent that have been brought into contact with each other may be stirred, may be shaken, or may be left to stand as appropriate.

By exposing the microorganism, and/or the culture liquid containing the microorganism to a high temperature condition during or before the extraction step as described above, the microorganism can be killed, and/or enzymes having an action to degrade MYCs in the microorganism can be inactivated, so that the amount of MYCs recovered can be improved. The high temperature condition to which the culture liquid is exposed may be, for example, not less than 40°C or not less than 50°C.

Therefore, the survival rate of the microorganism after the extraction step may be 0% to 20%, 0% to 10%, or 0% to 5% compared to that after the culture step. The survival rate is not limited as long as the survival rate after the extraction step is within this range. However, in cases where the survival rate of the microorganism is low also before the extraction step or during the extraction step, degradation of MYCs can be expected to be suppressed to improve the amount of MYCs recovered.

After the extraction step, the obtained extract may be subjected to removal of foreign substances, fractionation, purification, and the like by well-known methods.

The composition of the present disclosure preferably comprises MGGnol, MGGcol, and MGn in a total amount of preferably not less than 0.001% by weight based on the entire composition. The total amount may be more preferably not less than 0.01% by weight, still more preferably not less than 0.05% by weight.

Further, the composition of the present disclosure more preferably comprises MGGnol in an amount of not less than 0.0001% by weight based on the entire composition. The amount of MGGnol may be more preferably not less than 0.001% by weight, still more preferably not less than 0.005% by weight.

Further, of the total MYCs contained in the composition of the present disclosure, MGGnol may account for preferably not less than 10% by weight, more preferably not less than 20% by weight, still more preferably not less than 30% by weight.

By setting the content of MYCs within the above ranges, a desired effect can be readily achieved, and flexibility in the formulation design can be secured.

The above content can be appropriately adjusted in accordance with the route of administration described later and the mode of the composition in which the MYCs are to be contained.

The content of MYCs can be measured by a conventional method.

In general, MYCs have their ultraviolet-absorbing capacity due to structures containing cyclohexanone as a central ring. Therefore, confirmation of the presence of MYCs in a composition, and measurement of the amount and concentration of MYCs in a composition can be carried out using a light wavelength within the ultraviolet region. For example, these can be carried out by measuring the ultraviolet absorption of MYCs in the composition using a spectrophotometer (JP 5913988 B; Buscot et al., Mycol. Res, 95(6): 752-754 (1991)). The amount of MGGnol can be calculated by measuring the absorbance (ABS) at its maximum absorption wavelength, 310 nm, and performing calculation based on the molar absorption coefficient, 25,000 M⁻¹ cm⁻¹ (JP 5913988 B). MGGcol can be calculated by measuring the absorbance at its maximum absorption wavelength, 310 nm, and performing calculation based on the molar absorption coefficient, 25,000 M⁻¹ cm⁻¹ (N. Wada et al., Antioxidants, 4, 603-646 (2015)). Although MGn has been reported to have a maximum absorption wavelength of 310 nm (N. Wada et al., Antioxidants, 4, 603-646 (2015)), its molar absorption coefficient has not been reported. The amount of MGn can be calculated by using instead the molar absorption coefficient of mycosporine-glutamic acid (MGa), 20,900 M⁻¹ cm⁻¹ (N. Wada et al., Antioxidants, 4, 603-646 (2015)), which has almost the same molecular weight and a similar structure.

The composition of the present disclosure may also comprise MYCs other than the above-described MYCs, and even other MAAs. Examples of the other MAAs, including the other MYCs, may include compounds containing cyclohexanone or cyclohexenimine as a central ring; or compounds in which various functional groups such as amino acids are bound to the central ring.

More specific examples thereof include mycosporine-hydroxyglutamicol, gadusol, deoxygadusol, mycosporine-glycine, mycosporine-taurine, mycosporinealanine, mycosporine-serine, mycosporine-serinol, mycosporine-2-glycine, palythinol, palythenic acid, mycosporine-methylamine-threonine, mycosporine-glycine-valine, palythine, asterina-330, shinorine, porphyra-334, euhalothece-362, mycosporine-ornithine, mycosporine-lysine, mycosporine-glutamic acid-glycine, mycosporine-methylamine-serine, mycosporine-taurine, palythene, palythine-serine, palythine-serine-sulfate, and usujirene.

The composition of the present disclosure is capable of suppressing skin problems. The term "skin problems" in the present disclosure refers to erythema, inflammation, darkening/spots, and phenomena caused by oxidative stress, in skin.

One factor that allows the production of such an effect is that MYCs have an absorbing capacity for light over a wide range of wavelengths including midwavelength ultraviolet (UV-B: 280 to 320 nm) and long-wavelength ultraviolet (UV-A: 320 to 400 nm).

By absorption of UV-B by the MYCs, erythema and inflammation can be suppressed; pigmentation such as darkening and spots can be suppressed; and generation of reactive oxygen species (ROS) can be suppressed, as a result, to suppress oxidative stress. Carbonyl protein (CP) is a footprint of oxidative stress. In other words, generation of CP corresponds to a state with exposure to oxidative stress. Since wrinkles, spots, skin aging, and the like occur due to involvement of oxidative stress, these can be suppressed by suppression of oxidative stress. Generation of CP causes discoloration of skin to yellowish brown and skin dryness, and irradiation of CP with UV-A causes generation of reactive oxygen species, so that suppression of CP is of great significance.

Further, although the inventors of the present disclosure considered that the skin-problem-suppressing effect of MYCs was an effect due to absorption of UV-B by MYCs, particularly MGGnol, MGGcol, and MGn, the inventors surprisingly discovered that the above effect of the MYCs can also be obtained by suppression of oxidative stress through a pathway in which singlet oxygen, a type of reactive oxygen species generated by UV-A, is absorbed.

In addition, one factor contributing to the skin-problem-suppressing effect produced by the composition of the present disclosure is that MGG and MGn, which are MYCs, absorb singlet oxygen generated by UV-A, thereby suppressing oxidative stress.

Skin problems caused by UV-A continue over a longer period of time than those caused by UV-B, and the damage persists. Aging of the skin thus proceeds gradually. Therefore, there has hardly been an effective countermeasure against such skin problems. However, the present disclosure can provide a solution thereto.

Furthermore, another factor contributing to the skin-problem-suppressing effect produced by the composition of the present disclosure is that, even in the absence of ultraviolet irradiation (that is, regardless of the ultraviolet-absorbing capacity of MYCs), MYCs have an anti-inflammatory action and an active-oxygen-scavenging action, and therefore suppress skin inflammation and oxidative stress.

Thus, the composition of the present disclosure can suppress not only skin problems caused by ultraviolet irradiation, but also skin problems caused without ultraviolet irradiation.

Therefore, MYCs, particularly one, or two or more, selected from MGGnol, MGGcol, and MGn can serve as an active ingredient of a composition for suppressing a skin problem. The term "skin problem" herein refers to erythema, inflammation, darkening/spots, and phenomena caused by oxidative stress, in skin.

The Examples below show that, either in the presence or absence of UV-A and UV-B irradiation, MYCs suppressed the amount of the cytokine IL-1α produced and the amount of PGE₂ produced, which are indices of inflammation. PGE₂ reflects inflammation induced by oxidative stress such as ultraviolet irradiation, and is involved, together with nitric oxide radicals, in the generation of erythema; specifically, PGE₂ reddens the skin through a vasodilating action. Therefore, the Examples support the erythema-suppressing and anti-inflammatory actions.

The Examples below also show that, in a three-dimensional skin model irradiated with UV-B, MYCs suppressed the amount of CP. Accordingly, the Examples support the oxidative-stress-suppressing action.

Further, the Examples below show that, in a skin model irradiated with UV-B, MYCs suppressed darkening/spots, which supports such an effect.

Further, the Examples below show that, in a three-dimensional skin model or cells to which MYCs were applied, the amount of ROS was suppressed either in the presence or absence of UV-A and UV-B irradiation. This suggests both that the amount of ROS produced was suppressed and that the ROS produced was eliminated (removed) by the MYCs.

The present inventors discovered, for the first time, that MYCs can suppress skin problems regardless of the presence or absence of UV irradiation. Conventionally, MYCs and MAAs have been assumed, based on their chemical structures, to have an ultraviolet-absorbing capacity and to be capable of protecting skin by ultraviolet protection. However, the fact that they can suppress various skin problems even in a state without exposure to ultraviolet radiation has not been inferred from the prior art at all.

In the present description, the term "suppression" of a skin problem may include prevention of a skin problem that will occur in the future and prevention of exacerbation of a skin problem that has already occurred. The expression "prevention of exacerbation of a skin problem that has already occurred" may include preventing the skin problem from becoming more severe and delaying an increase in the severity of the skin problem.

Furthermore, since one, or two or more, selected particularly from MGGnol, MGGcol, and MGn can suppress the amount of ROS as described above, they can also serve as an active ingredient of a composition for suppressing reactive oxygen species.

In the present description, the term "suppression" of reactive oxygen species may include a reduction in the amount of ROS generated and elimination (removal) of ROS that has been generated.

The intended use of the composition of the present disclosure may be either therapeutic use or non-therapeutic use.

The term "non-therapeutic use" refers to an act that does not include medical practice, that is, a concept that does not include an act of treatment to a human body. Examples of the non-therapeutic use include health promotion and cosmetic acts.

In cases where the composition of the present disclosure is used for a non-therapeutic purpose, the composition is administered to a healthy individual. In cases where the composition of the present disclosure is used for a non-therapeutic purpose, the composition may be used for suppressing skin problems and/or suppressing reactive oxygen species in a healthy individual. Further, in cases where the composition of the present disclosure is used for a non-therapeutic purpose, the composition may be used for preventing diseases and symptoms caused by inflammation, erythema, skin darkening, spot formation, oxidative stress, or the like due to exposure to ultraviolet.

In cases where the composition of the present disclosure is used for a therapeutic purpose, the composition may be administered to a non-healthy individual. Examples of such a non-healthy individual include individuals with a disease or symptom caused by inflammation, erythema, skin darkening, spot formation, oxidative stress, or the like due to ultraviolet exposure, and the composition may be used for suppressing or alleviating these diseases and symptoms.

Examples of the disease or symptom caused by inflammation, erythema, skin darkening, spot formation, oxidative stress, or the like due to ultraviolet exposure include skin cancer, and autoimmune diseases such as systemic lupus erythematosus and Sjögren's syndrome (see, for example, Non-Patent Document 2, T. Ogura et al., Jpn. J. Clin. Immunol., 37(1), 25-32 (2014); Yoichiro Hamasaki, J Jpn. Org. Clin. Dermatol., 33(4), 483-491 (2016)). Skin cancer is considered to be caused by oxidative stress due to long-term ultraviolet exposure and the resulting decrease in immune function and so-called photoaging. Specific examples of skin cancer include actinic keratosis, squamous cell carcinoma, basal cell carcinoma, and melanoma. Autoimmune diseases are multifactorial diseases, and both genetic predisposition and environmental factors are intricately involved in their onset. When oxidative stress due to environmental factors such as ultraviolet exposure becomes excessive, inflammation, tissue damage due to apoptosis induction, induction of autoimmune responses, and the like may occur to form autoimmune pathology. Systemic lupus erythematosus and Sjögren's syndrome are also known as major autoimmune diseases, and elimination of undesirable phenomena caused by ultraviolet exposure is important for avoiding these diseases.

Another aspect of the present invention is use of MYCs in the production of a composition for suppressing a skin problem and/or a composition for suppressing reactive oxygen species.

Another aspect of the present invention is use of MYCs in suppressing a skin problem and/or suppressing reactive oxygen species.

Another aspect of the present invention is MYCs for use in suppressing a skin problem and/or suppressing reactive oxygen species.

Another aspect of the present invention is a method of suppressing a skin problem and/or suppressing reactive oxygen species, the method including administering MYCs to an animal (causing the animal to ingest MYCs).

The timing of administration (ingestion) of the composition of the present disclosure is not particularly limited and may be appropriately selected according to conditions of the subject to whom the composition is to be administered.

The dosing (ingestion) amount of the composition of the present disclosure may be appropriately selected according to the age, sex, conditions, and other factors of the subject to whom the composition is to be administered (the subject who ingests the composition).

The dosing (ingestion) amount of the composition of the present disclosure is not particularly limited in terms of the dosing (ingestion) amount of MYCs. However, from the viewpoint of the desired effect and safety, for example, when the composition is transdermally administered in the form of the later-described topical skin composition, the total amount of MGGnol, MGGcol, and MGn per skin surface area in an adult is preferably not less than 0.1 µg/cm²/day, and may be more preferably not less than 0.2 µg/cm²/day, not less than 0.4 µg/cm²/day, not less than 0.8 µg/cm²/day, not less than 1.6 µg/cm²/day, not less than 3.2 µg/cm²/day, or not less than 6.4 µg/cm²/day. There is no particular upper limit of the dose.

The dose may also be appropriately adjusted depending on the skin problem to be targeted. For example, for the purpose of suppressing erythema and/or inflammation in skin, the total amount of MGGnol, MGGcol, and MGn may not be less than 0.1 µg/cm²/day. For the purpose of suppressing darkening and/or spots in skin, the total amount of MGGnol, MGGcol, and MGn may not be less than 0.5 µg/cm²/day. For the purpose of suppressing phenomena caused by oxidative stress in skin, the total amount of MGGnol, MGGcol, and MGn may not be less than 0.1 µg/cm²/day.

Regardless of the dosing (ingestion) amount and the dosing (ingestion) period, the composition of the present disclosure may be administered once per day or divided into a plurality of times per day. The composition may be given by single administration (ingestion), or may be given by continuous or intermittent administration (ingestion) for a period of not less than several weeks or not less than several months.

The subject to whom the composition of the present disclosure is to be administered (the subject who ingests the composition) is not particularly limited, and examples of the subject include mammals. Examples of the mammals include humans, dogs, and cats. In particular, examples of the mammals include humans. The humans may be those of any age including infants, toddlers, children, adults, middle-aged persons, and elderly persons. The sex is not particularly limited. Further, since the UV-B-absorbing capacity of MYCs described above is achieved as long as the MYCs are allowed to be present on the cell surface by the administration, there is no limitation regarding the skin conditions and the skin type of the subject to whom the composition is to be administered.

The administration (ingestion) route of the composition of the present disclosure is not particularly limited, and may be, for example, transdermal administration, oral administration, transnasal administration, inhalation, or intravenous injection. The administration (ingestion) route is usually transdermal administration. The "administration" may be replaced with "ingestion" herein.

In the case of administration (ingestion) by transdermal administration, the composition is preferably in the form of a topical skin composition.

The mode of the topical skin composition is not limited as long as the composition is topically applied to skin. Preferred examples of the mode of the composition include cosmetics, quasi-drugs, and pharmaceuticals. Cosmetics are more preferred. Sunscreen cosmetics are especially preferred. In cases where the mode of the composition of the present disclosure is a cosmetic, the composition is used for the non-therapeutic purpose described above. In cases where the mode of the composition of the present disclosure is a quasi-drug or a pharmaceutical, the composition is used for the therapeutic purpose described above.

The site to which the topical skin composition is applied is not particularly limited, and is usually a facial surface, limb, neck, or decollete.

Examples of the formulation of the topical skin composition include, but are not limited to, lotion formulations, emulsion formulations such as milky lotions and creams, oil formulations, gel formulations, packs, and cleansing agents.

Regarding the mode of the topical skin composition, the composition may be of either a leave-on type or a rinse-off type.

In cases where the mode of the composition of the present disclosure is a topical skin composition, not only MYCs, but also arbitrary components that are usually used for formulating cosmetics, quasi-drugs, pharmaceuticals, and the like may be blended for the production of the composition.

Examples of such arbitrary components to be blended include hydrocarbons such as squalane, petrolatum, and microcrystalline wax; esters such as jojoba oil, carnauba wax, and octyldodecyl oleate; triglycerides such as olive oil, tallow, and coconut oil; fatty acids such as stearic acid, oleic acid, and retinoic acid; higher alcohols such as oleyl alcohol, stearyl alcohol, and octyldodecanol; anionic surfactants such as sulfosuccinic acid esters and sodium polyoxyethylene alkyl sulfate; amphoteric surfactants such as alkyl betaine salts; cationic surfactants such as dialkylammonium salts; nonionic surfactants such as sorbitan fatty acid esters, fatty acid monoglycerides, polyoxyethylene adducts thereof, polyoxyethylene alkyl ethers, and polyoxyethylene fatty acid esters; polyols such as polyethylene glycol, glycerin, and 1,3-butanediol; thickeners/gelling agents; antioxidants; ultraviolet-scattering agents; colorants; preservatives; and powders.

Unless otherwise defined, all technical terms and scientific terms used herein have the same meanings as those generally understood by those skilled in the art to which the present disclosure belongs. Any of the methods and materials similar or equivalent to the methods and materials described herein can be used in the practice or testing of the present disclosure, and representative exemplary methods and materials are described herein.

It is understood that, when a range of values is provided, the individual values between the upper limit and the lower limit of the range (to the tenth of the unit of the lower limit unless the context clearly dictates otherwise), and any other described values or intervening values within the described range are encompassed within the scope of the invention presented in the present disclosure. The upper and lower limits of a narrower range thereof may be independently included in the narrower range. They are also encompassed within the scope of the invention presented in the present disclosure, and subject to any specifically excluded limit in the described range. In cases where a described range includes one or both of the limits, ranges excluding one or both of those included limits are also included in the invention presented in the present disclosure.

The present disclosure is not limited to the specific modes described. Since the scope of the invention presented in the present disclosure is considered to be limited only by the appended claims, it should also be understood that the terminology used herein is merely for the purpose of describing particular modes, and is not intended to be limiting.

As will be apparent to those skilled in the art upon reading this disclosure, each of the individual modes described herein has discrete components and features that may be readily separated from or combined with features of several other modes without departing from the scope and the spirit of the invention of the present disclosure. Any cited method may be implemented in the order of the cited events or in any other order that is logically possible and consistent.

All publications and patents cited herein are hereby incorporated by reference as if each individual publication or patent were specifically and individually indicated to be incorporated by reference, and the publications are incorporated herein by reference to disclose and describe the methods and/or materials with which the publications are cited in connection. The citation of any publication is for its disclosure prior to the filing date, and should not be construed as an admission that, by virtue of prior invention, the invention described in the present disclosure is not entitled to antedate such publication. Further, the dates of publication provided may be different from the actual publication dates, and the actual publication dates may need to be individually confirmed.

The following Examples are given for the purpose of exemplifying various modes of the present disclosure, and are not intended to limit the present disclosure in any fashion. Those skilled in the art will readily appreciate that the present disclosure is well adapted to carry out the mentioned objects to achieve the final goal and the advantages mentioned, and also well adapted to carry out the inherent objects of the present description to achieve the final goal and the advantages. The present Examples, along with the methods described herein, are representative modes at present. They are exemplary, and not intended to limit the scope of the present disclosure. Modifications and other uses encompassed within the spirit of the present disclosure as defined by the claims will occur to those skilled in the art.

### EXAMPLES

The invention presented in the present disclosure is described below by way of Examples, but the invention presented in the present disclosure is not limited by these Examples. Unless otherwise specified, the units are on a weight basis.

### <Reference Example> Preparation of MYC Samples

By the method described above, microorganisms that produce MGGnol, MGGcol, and/or MGn (*Aureobasidium pullulans* and/or *Hormonema macrosporum*) were cultured, and MYCs were extracted from the resulting cultured fungal cells.

The concentrations of MYCs in the resulting extract were measured by HPLC. Specifically, an HPLC system manufactured by Shimadzu Corporation was used with the following: a Lichrospher (registered trademark) 100RP-18 column (particle size, 5 µm; 4mm (inner diameter) × 250 mm (length); manufactured by Merck) equipped with a guard column LiChrospher (registered trademark) 100 RP-18 (5 µm) LiChroCART (registered trademark) 4-4; a mobile phase (aqueous solution of 0.5% methanol and 0.2% acetic acid); a flow rate of 0.7 mL/min.; a column oven temperature of 35°C; an injection volume of 10 µL; and measurement at 250 nm to 400 nm using a PDA inspection device. A peak with a maximum absorption wavelength near 310 nm was found, and this was regarded as the peak of MAAs. Quantification was carried out at a detection wavelength of 310 nm.

Three types of MYC samples were prepared/obtained. Table 1 shows the MGGnol, MGGcol, and MGn contents in those samples.

### [Table 1]

**Table 1**

| MYCs sample | MYCs contents (%) | | |
|---|---|---|---|
| | MGn | MGGnol | MGGcol |
| MGG-K | 37 | 60 | 4 |
| MGG-EX | - | 97 | 3 |
| MGG-E | - | 80 | 20 |

### <Example 1> Study of Anti-Inflammatory Action and Erythema-Suppressing Action

### (1) Method of Culturing a Three-Dimensional Skin Model and Preliminary Study of the Ultraviolet Irradiation Dose

In order to evaluate the response of a three-dimensional skin model to UV-B, an irradiation dose that does not cause cell death was investigated. Further, comparison was made with results obtained using skin cells of a prior art (Patent Document 4).

Sample MGG-E was prepared at an MYC concentration of 0.07, 0.14, or 0.35% by weight to provide an MYC solution.

LabCyte EPI-MODEL 24 (Japan Tissue Engineering Co., Ltd.) was used as a three-dimensional skin model. Using the supplied assay medium, the model was acclimated in a 24-well plate at a culture temperature of 37°C at 5% CO₂ for 24 hours. Ultraviolet irradiation was performed using an ultraviolet lamp (TL 20W/12 RS, manufactured by PHILIPS Lighting). The irradiation intensity of the ultraviolet lamp was measured using a UVX Radiometer manufactured by Analytik Jena. The irradiation time was adjusted such that an irradiation dose of 400 mJ/cm² or 600 mJ/cm² was achieved.

After the acclimation, 30 µL of PBS(-) or the MYC solution was applied to the three-dimensional skin model in one well from the stratum corneum side of LabCyte EPI-MODEL 24, and culture was performed at a culture temperature of 37°C at 5% CO₂ for 24 hours. Thereafter, UV-B irradiation was performed at 400 or 600 mJ/cm². Twenty-four hours after the irradiation, PBS(-) or the MYC solution on the stratum corneum was removed by washing with 500 µL of PBS(-), and the cell viability was measured by the Alamar Blue Assay. It should be noted that, since the amount of MYCs in 30 µL of the 0.07% MYC solution was 2.1 µg, and the area of the well was 0.32 cm², the amount of MYCs applied was 6.6 µg/cm².

The cells survived at an irradiation dose of either 400 or 600 mJ/cm² in the presence of the MYCs.

### (2) Study of Anti-Inflammatory Action and Erythema-Suppressing Action

IL-1α is always retained in cells, and easily leaks out of the cells due to stimulation by ultraviolet irradiation. Since IL-1α can be detected rapidly and in a large amount, it can be widely evaluated as an inflammatory mediator (T. Hirano, et al., Dermatol 106,1102-1107(1996)). In addition, as described above, PGE₂ is involved in generation of erythema, and is an index of inflammation induced by oxidative stress such as ultraviolet irradiation (Non-Patent Document 2).

A three-dimensional skin model was provided as described above. As studied in (1), an irradiation dose of 600 mJ/cm² did not decrease the cell viability. Therefore, 600 mJ/cm², which provides a greater influence of light irradiation, was selected for testing. The amount of PGE₂ that flowed out into the medium 24 hours after the irradiation was measured using a Prostaglandin E2, EIA Monoclonal Kit (manufactured by Cayman), and the amount of IL-1α was measured using a Human IL-1 alpha/IL-1F1 Quantikine ELISA Kit (manufactured by R&D).

Table 2 shows the amounts of IL-1α and PGE₂ produced per three-dimensional skin model in one well, respectively.

As a result of irradiation at 600 mJ/cm², IL-1α increased from 6 pg/3D to 11 pg/3D. Similarly, PGE₂ increased from 25 to 47 pg/3D. Both IL-1α and PGE₂ were found to increase about 2-fold under the condition of 600 mJ/cm². When 30 µL of 0.07% MYC solution or 0.14% MYC solution prepared from Sample MGG-E in (1) was applied to one well from the stratum corneum side, IL-1α and PGE₂ decreased in a manner dependent on the MYC concentration.

Since the addition of 0.14% MYCs resulted in lower IL-1α than that of the control, the MYCs were found to have an anti-inflammatory action regardless of UV-B irradiation.

### [Table 2]

**Table 2**

| Test group | UV-B (mJ/cm²) | MYCs (%) | IL-1α production (pg/3D) | PGE₂ production (pd/3D) |
|---|---|---|---|---|
| Control | 0 | 0 | 6 | 25 |
| Test group 1 | 600 | 0.00 | 11 | 47 |
| Test group 2 | | 0.07 | 8 | 39 |
| Test group 3 | | 0.14 | 4 | 37 |

### <Example 2> Study of Skin-Darkening-Suppressing Action

Human skin excised from a Caucasian female of 39 years old with a BMI of 28 was thinly sliced to a diameter of 14 mm (surface area: 1.5 cm²) and a thickness of 400±100 µm, and subjected to an experiment. The skin was mounted on Intercell, and then placed in a well containing a medium, followed by performing acclimation culture at a culture temperature of 37°C at 5% CO₂ for 24 hours.

An MYC solution was prepared from MGG-K by adjusting the MYC content to 0.7% with PBS(-). The amount of the MYC solution applied, in terms of the amount of MYCs, was set to 22.7 µg/cm² per skin surface area.

Test groups were set as follows: control (no irradiation during the entire period); Test Group 4, with UV-B irradiation (irradiation during the entire period); Test Group 5, with addition of MYCs before and after UV-B irradiation; Test Group 6, with addition during UV-B irradiation; and Test Group 7, with addition before, during, and after UV-B irradiation (addition during the entire period) (see Table 3). The sample number in each test group was set to 3.

A silicone washer was placed on the skin, and 50 µL of PBS(-) or the MYC solution was added. Twenty-four hours later, the PBS(-) or the MYC solution was removed, and washing was performed three times using 1 mL of PBS(-). The PBS(-) remaining on the Intercell or the skin surface was removed. Fresh PBS(-) or MYC solution was dropped into the silicone washer, and the skin was transferred to a plate containing PBS(-). Thereafter, UV-B irradiation was performed at 300 mJ/cm² using an ultraviolet lamp (TL 20W/12 RS, manufactured by PHILIPS Lighting). After the irradiation, the PBS(-) or the MYC solution was removed. Thereafter, the skin was returned to a plate containing a medium, and then cultured. The same operation was repeated for 2 days. UV-B irradiation was thus performed a total of three times. After the final irradiation, culture was continued while visually confirming darkening of the skin. The skin was recovered from the Intercell 72 hours after the third irradiation, and a photograph was taken together with a color chart (Casmatch; manufactured by Bear Medic).

With reference to a method of evaluating skin darkening using the brightness index L* (Gerg. G. Hillebrand, et al., J. Dermatol. Sci 27, S42-S52 (2001)), the captured image was subjected to image correction according to the manufacturer's instruction of Casmatch, and the L* value was calculated using Photoshop. The average L* value within a certain area was calculated for three samples per test group.

### [Table 3]

**Table 3**

| Time (hr) | 0-24 | 24 | 24-48 | 48 | 48-72 | 72 | 72-120 |
|---|---|---|---|---|---|---|---|
| UV-B irradiation | | 1st | | 2nd | | 3rd | |
| Control | PBS | No irradiation PBS | PBS | No irradiation PBS | PBS | No irradiation PBS | PBS |
| Test group 4 | PBS | PBS | PBS | PBS | PBS | PBS | PBS |
| Test group 5 | MYCs | PBS | MYCs | PBS | MYCs | PBS | MYCs |
| Test group 6 | PBS | MYCs | PBS | MYCs | PBS | MYCs | PBS |
| Test group 7 | MYCs | MYCs | MYCs | MYCs | MYCs | MYCs | MYCs |

Table 4 shows the results.

A decrease in the brightness index L* indicates occurrence of skin darkening.

In Test Groups 4 and 5, UV-B irradiation resulted in a significant decrease in the brightness (L* value) by about 10%. In other words, the skin darkening occurred.

On the other hand, in Test Groups 6 and 7, the L* value decreased by about 2% compared to the control, but the difference was not significant. It was found that darkening of the skin was suppressed by the application of 50 µL of the 0.7% MYC solution to the skin during the irradiation. In the case where MYC addition before the first UV-B irradiation was followed by washing, irradiation, and then MYC addition, no darkening-suppressing effect was found. Thus, it was suggested that suppression of darkening requires the presence of the MYCs on the skin during the ultraviolet irradiation.

### [Table 4]

**Table 4**

| Test group | UV-B irradiation | L* value | | | Average | S.D. |
|---|---|---|---|---|---|---|
| Control | No irradiation | 202.2 | 212.0 | 206.8 | 207.0 | 4.9 |
| Test group 4 | Irradiation | 176.4 | 189.0 | 185.4 | 183.6 | 6.5 |
| Test group 5 | | 197.0 | 185.6 | 184.8 | 189.1 | 6.8 |
| Test group 6 | | 198.2 | 206.4 | 207.9 | 204.2 | 5.2 |
| Test group7 | | 205.5 | 198.2 | 201.9 | 201.9 | 3.7 |

### <Example 3> Study of Suppressing Action on Skin Damage Caused by Active Oxygen

Evaluation was carried out with reference to a method by Katsuyama et al. (Yushi Katsuyama et al., Journal of Japanese Cosmetic Science Society, 39, 89-94 (2015)).

LabCyte EPI-MODEL 24 (Japan Tissue Engineering Co., Ltd.) was used as a three-dimensional skin model. Using the supplied assay medium, the model was acclimated in a 24-well plate at a culture temperature of 37°C at 5% CO₂ for 24 hours. Thereafter, 30 µL/well of PBS(-), Sample MGG-K (after adjustment of the concentration to 0.15% MYCs), Sample MGG-EX (after adjustment of the concentration to 0.15% MYCs), or Sample MGG-E (after adjustment of the concentration to 0.14% MYCs) was applied from the stratum corneum side of LabCyte EPI-MODEL 24, and culture was performed at a culture temperature of 37°C at 5% CO₂ for 24 hours. Twenty-four hours later, the PBS(-) or each MYC solution on the stratum corneum was removed, and washing was performed with PBS(-). After application of 30 µL of freshly prepared PBS(-) or each MYC solution from the stratum corneum side, UV-B irradiation was performed at 600 mJ/cm² using a UV-B lamp (TL 20W/12 RS) (PHILIPS Lighting). The sample number in each test group was set to 4.

After the UV-B irradiation, the PBS(-), the MGG-K solution, or the MGG-EX solution on the stratum corneum was removed, and the stratum corneum surface was washed with PBS(-). The ROS level in the epidermal tissue was evaluated by detection of chemiluminescence using the 2-methyl-6-(4-methoxyphenyl)-3,7-dihydroimidazo[1,2-a]pyrazin-3-one hydrochloride (MCLA) reagent (Tokyo Chemical Industry Co., Ltd.) As a detector, Night OWL (registered trademark) (manufactured by Berthold Technologies) was used. Image processing was carried out using Image J.

The results are shown in Table 5. A higher gray value indicates a larger amount of ROS. As reported in many documents, the amount of ROS increased by 4% in the case where UV-B irradiation was performed. By the addition of the MYCs, the amount of ROS decreased by about 10% as compared to the case where UV-B irradiation was performed. Further, surprisingly, the amount of ROS in the control, that is, the amount of ROS constantly generated by biological reaction even without UV-B irradiation, decreased by the addition of the MYCs.

### [Table 5]

**Table 5**

| Test group | UV-B irradiation | Treatment | MYCs addition (%) | Average of gray value (%) |
|---|---|---|---|---|
| Control | No irradiation | PBS | - | 100 |
| Test group8 | Irradiation | PBS | - | 104 |
| Test group9 | | MGG-K | 0.15 | 95 |
| Test group10 | | MGG-EX | 0.15 | 95 |
| Test group11 | | MGG-E | 0.14 | 74 |

### <Example 4> Study of Suppressive Action on Carbonyl Protein Generated by Ultraviolet Exposure

LabCyte EPI-MODEL 24 (Japan Tissue Engineering Co., Ltd.) was used as a three-dimensional skin model. Using the supplied assay medium, the model was acclimated in a 24-well plate at a culture temperature of 37°C at 5% CO₂ for 24 hours. Thereafter, 30 µL/well of PBS(-), Sample MGG-K (after adjustment of the concentration to 0.15% MYCs), Sample MGG-EX (after adjustment of the concentration to 0.15% MYCs), or Sample MGG-E (after adjustment of the concentration to 0.14% MYCs) was applied from the stratum corneum side of LabCyte EPI-MODEL 24, and culture was performed at a culture temperature of 37°C at 5% CO₂ for 24 hours. Twenty-four hours later, the PBS(-) or each MYC solution on the stratum corneum was removed, and washing was performed with PBS(-). After application of 30 µL of freshly prepared PBS(-) or each MYC solution from the stratum corneum side, UV-B irradiation was performed at 600 mJ/cm² using a UV-B lamp (TL 20W/12 RS) (PHILIPS Lighting). The sample number in each test group was set to 4.

After the UV-B irradiation, the PBS(-) or each MYC solution on the stratum corneum was removed, and the stratum corneum surface was washed with PBS(-). After application of 30 µL of freshly prepared PBS(-) or each MYC solution from the stratum corneum side, culture was performed for 24 hours. Thereafter, the PBS(-), the MGG-K solution, or the MGG-EX solution on the stratum corneum was removed, and the stratum corneum surface was washed with PBS(-).

Epidermal tissue of the three-dimensional cultured skin model was removed from a culture cup, and a frozen block was prepared using O.C.T. Compound (manufactured by Sakura Finetek Japan Co., Ltd.), followed by preparation of thin sections.

Carbonyl protein (CP) in the thin sections was detected using fluorescein-5-thiosemicarbazide (manufactured by Invitrogen) (see T. Mizutani, et al., Journal of Japanese Cosmetic Science Society, 41, 101-105 (2017)). Further, the CP level was quantified by fluorescence image analysis. As image analysis software, Corneocytemetry 2 (manufactured by CIEL Co., Ltd.) was used.

Table 6 shows the relative amount of CP with respect to the control. CP increased by 1.27-fold due to UV-B irradiation. By the addition of the MYCs, the increase in CP was suppressed.

### [Table 6]

**Table 6**

| Test group | UV-B irradiation | Treatment | Relative amount of CP |
|---|---|---|---|
| Control | No irradiation | PBS | 100 |
| Test group12 | Irradiation | PBS | 127 |
| Test group13 | | MGG-K | 96 |
| Test group14 | | MGG-EX | 95 |
| Test group15 | | MGG-E | 102 |

### <Example 5> Study of Amount of Intracellular ROS as Observed for Cases with or without UV-A Irradiation

As cells, normal human epidermal keratinocytes (manufactured by Kurabo Industries Ltd.) were used. The cells were cultured using a standard culture medium (HuMedia-KG2). For ultraviolet irradiation, an ultraviolet UV-A lamp (FL20SBLB 20W, manufactured by Toshiba Corporation) was used. An MYC solution prepared by diluting Sample MGG-EX with PBS(-) was added to the cells, and the cells were left to stand for 24 hours. Thereafter, the MYC solution was added again together with riboflavin. Under the coexistence of MYCs and riboflavin, UV-A irradiation was performed at 6 J/cm², and the intracellular ROS level immediately after the irradiation was detected using a cell-permeable redoxsensitive fluorescent probe, DCFDA (2,7-dichlorofluoroscin diacetate). Further, the amount of cellular protein was quantified using a BCA Protein Assay Kit (Thermo Scientific (registered trademark)). The sample number in each test group was set to 3.

The results are shown in Fig. 1, Table 7 (without UV-A irradiation), and 8 (without UV-A irradiation). The amount of protein was used as an indicator of the number of cells. As shown in the tables, no change in the amount of protein was found as a result of the MYC application and the UV-A irradiation. The amount of intracellular ROS produced without UV-A irradiation is shown. As the MYC concentration increased, ROS decreased. ROS significantly decreased even at an MYC concentration of as low as 0.006%. With 0.05% MYCs, ROS decreased by half compared to the control.

Further, when UV-A irradiation was carried out, ROS increased 1.5-fold even in the control due to the UV-A irradiation. When UV-A irradiation was carried out, a significant decrease in ROS was achieved at a lower MYC concentration (0.003%) compared to the case without irradiation. As the MYC concentration increased, ROS decreased. With 0.05% MYCs, ROS decreased to about one third compared to the control for the case with UV-A irradiation. As a result of the application of the MYCs, intracellular ROS decreased in a manner dependent on the MYC concentration regardless of whether or not UV-A irradiation was carried out.

### [Table 7]

**Table 7**

| Test group | MYC(%) | Protein(µg) | | Intracellular ROS/µg protein | | |
|---|---|---|---|---|---|---|
| | | Average | p vs. control | Average | p vs. control | |
| Control | 0 | 29.5 | 1.000 | 1183 | 1.000 | |
| Test group16 | 0.002 | 29.8 | 0.788 | 1063 | 0.363 | |
| Test group17 | 0.003 | 30.2 | 0.369 | 979 | 0.127 | |
| Test group18 | 0.006 | 31.0 | 0.132 | 896 | 0.041 | p<0.05 |
| Test group19 | 0.013 | 30.7 | 0.423 | 794 | 0.022 | p<0.05 |
| Test group20 | 0.025 | 30.7 | 0.425 | 634 | 0.005 | p<0.01 |
| Test group21 | 0.05 | 30.3 | 0.376 | 533 | 0.003 | p<0.01 |

### [Table 8]

**Table 8**

| Test group | MYC(%) | Protein(µg) | | Intracellular ROS/µg protein | | | | |
|---|---|---|---|---|---|---|---|---|
| | | Average | p vs. control | Average | p vs. control | | p vs. No irradiation | |
| Control | 0 | 29.8 | 1.000 | 1843 | 1.000 | | 0.003 | p<0.01 |
| Test group22 | 0.002 | 29.1 | 0.273 | 1811 | 0.671 | | 0.001 | p<0.01 |
| Test group23 | 0.003 | 30.2 | 0.515 | 1602 | 0.018 | p<0.01 | 0.001 | p<0.01 |
| Test group24 | 0.006 | 29.9 | 0.879 | 1351 | 0.002 | p<0.01 | 0.001 | p<0.01 |
| Test group25 | 0.013 | 30.3 | 0.520 | 1140 | 0.001 | p<0.01 | 0.008 | p<0.01 |
| Test group26 | 0.025 | 30.2 | 0.701 | 903 | 0.000 | p<0.01 | 0.008 | p<0.01 |
| Test group27 | 0.05 | 30.6 | 0.467 | 644 | 0.000 | p<0.01 | 0.042 | p<0.05 |

## Claims

1. A composition for suppressing a skin problem, the composition comprising a mycosporine (MYC).

2. A composition for suppressing reactive oxygen species, the composition comprising a mycosporine (MYC).

3. The composition according to claim 1 or 2, wherein the MYC comprises one, or two or more, selected from mycosporine-glutaminol-glucoside (MGGnol), mycosporine-glutamicol-glucoside (MGGcol), and mycosporine-glutamine (MGn).

4. The composition according to claim 3, wherein the MYC comprises at least MGGnol and MGGcol.

5. The composition according to claim 3, comprising MGGnol, MGGcol, and MGn in a total amount of not less than 0.001% by weight based on the entire composition.

6. The composition according to claim 3, which is used by application to skin in a total amount of MGGnol, MGGcol, and MGn of not less than 0.1 µg/cm²/day.

7. The composition according to claim 1, wherein the skin problem is one or more selected from erythema, inflammation, darkening/spots, and phenomena caused by oxidative stress, in skin.

8. The composition according to claim 1, wherein the skin problem is a skin problem caused by ultraviolet irradiation, or a skin problem caused without ultraviolet irradiation.

9. The composition according to claim 1 or 2, which is a topical skin composition.

10. The composition according to claim 9, which is used by application to skin in a total amount of MGGnol, MGGcol, and MGn of not less than 0.1 µg/cm²/day.

11. The composition according to claim 9, which is for suppressing erythema and/or inflammation in skin and is used by application to skin in a total amount of MGGnol, MGGcol, and MGn of not less than 0.1 µg/cm²/day.

12. The composition according to claim 9, which is for suppressing darkening and/or spots in skin and is used by application to skin in a total amount of MGGnol, MGGcol, and MGn of not less than 0.5 µg/cm²/day.

13. The composition according to claim 9, which is for suppressing a phenomenon caused by oxidative stress in skin and is used by application to skin in a total amount of MGGnol, MGGcol, and MGn of not less than 0.1 µg/cm²/day.

14. The composition according to claim 9, which is a sunscreen cosmetic.
